(19) Europäisches Patentamt
European Patent Office
Office européen des brevets

(11) EP 4 420 516 A1

(12) EUROPEAN PATENT APPLICATION
published in accordance with Art. 153(4) EPC

(43) Date of publication:
**28.08.2024 Bulletin 2024/35**

(21) Application number: **22883809.0**

(22) Date of filing: **28.09.2022**

(51) International Patent Classification (IPC):
***A01N 33/12*** (2006.01) ***A01P 1/00*** (2006.01)
***A01P 3/00*** (2006.01) ***C12Q 1/18*** (2006.01)
***C08F 212/08*** (2006.01) ***C08F 220/44*** (2006.01)
***C08F 236/06*** (2006.01) ***C08L 25/06*** (2006.01)
***C08L 25/10*** (2006.01) ***C08L 9/00*** (2006.01)

(52) Cooperative Patent Classification (CPC):
**A01N 33/12; A01P 1/00; A01P 3/00; C08F 212/08; C08F 220/44; C08F 236/06; C08L 9/00; C08L 25/06; C08L 25/10; C12Q 1/18**

(86) International application number:
**PCT/KR2022/014549**

(87) International publication number:
**WO 2023/068585 (27.04.2023 Gazette 2023/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.10.2021 KR 20210141414**
**19.09.2022 KR 20220118143**

(71) Applicant: **LG Chem, Ltd.**
**Seoul 07336 (KR)**

(72) Inventors:
- **LEE, Seungmo**
  **Daejeon 34122 (KR)**
- **BAEK, Leehyeon**
  **Daejeon 34122 (KR)**
- **LEE, Jeong Yun**
  **Daejeon 34122 (KR)**
- **LEE, Hwanhee**
  **Daejeon 34122 (KR)**
- **CHOI, Doowhan**
  **Daejeon 34122 (KR)**
- **HUR, Yoon Hyung**
  **Daejeon 34122 (KR)**
- **LEE, Jiyoung**
  **Daejeon 34122 (KR)**
- **CHOI, Hyungsam**
  **Daejeon 34122 (KR)**

(74) Representative: **Goddar, Heinz J. et al**
**Boehmert & Boehmert**
**Anwaltspartnerschaft mbB**
**Pettenkoferstrasse 22**
**80336 München (DE)**

# (54) ANTIBACTERIAL COMPOSITION

(57) An antibacterial composition including a compound represented by the disclosed formula 1 and having an antibacterial activity of 80% or more as measured by the disclosed method 1, against at least one strain of gram-positive bacteria, gram-negative bacteria, and fungus. Since the antibacterial composition includes a hydrophilic functional group and a hydrophobic functional group at the same time, it is advantageous for imparting antibacterial properties.

EP 4 420 516 A1

## Description

[Technical Field]

The present invention relates to an antibacterial composition.

< CROSS-REFERENCE TO RELATED APPLICATIONS>

This application claims priorities to and the benefits of Korean Patent Application No. 10-2021-0141414 filed in the Korean Intellectual Property Office on October 21, 2021, and Korean Patent Application No. 10-2022-0118143 filed in the Korean Intellectual Property Office on September 19, 2022, the entire contents of which are incorporated herein by reference.

[Background Art]

Recently, high antibacterial properties are required for various products such as household products and hygiene products.

Depending on the material of a product requiring antibacterial properties or the state of final use, the required degree of antibacterial properties and the material requirements for imparting antibacterial properties are different. For example, depending on the usage of the antibacterial material applied to a product or the material used together, the properties of the material for imparting the antibacterial property and the degree of the antibacterial property are different.

Accordingly, it is necessary to develop an antibacterial material suitable for application to each of various products.

[Detailed Description of the Invention]

[Technical Problem]

An embodiment of the present invention is to provide an antibacterial composition advantageous for imparting antibacterial properties by having hydrophilicity and hydrophobicity.

[Technical Solution]

An embodiment of the present invention provides an antibacterial composition including a compound represented by formula 1 below and having an antibacterial activity of 80% or more, as measured by method 1 below, against at least one strain of gram-positive bacteria, gram-negative bacteria, and fungus:

[Formula 1]

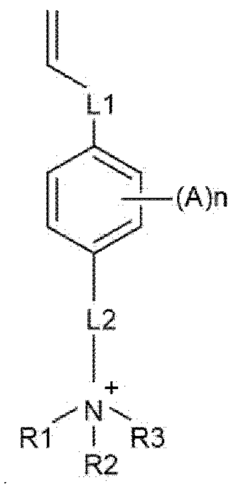

wherein,

L1 and L2 are the same as or different from each other, and each independently a direct bond; a substituted or unsubstituted alkylene group having 1 to 4 carbon atoms; or a substituted or unsubstituted arylene group having 6 to 30 carbon atoms,
A is hydrogen; or a substituted or unsubstituted alkyl group having 1 to 3 carbon atoms,
n is an integer of 0 to 4,
R1 and R2 are the same as or different from each other, and each independently a substituted or unsubstituted alkyl group having 1 to 20 carbon atoms, and at least one of R1 and R2 is a substituted or unsubstituted alkyl group having 1 to 10 carbon atoms, and
R3 is an alkyl group having 4 to 20 carbon atoms, wherein when n is 2 or more, As having n of 2 or more are the same as or different from each other, and
[Method 1]
after putting 25 mL of a broth-type medium (Nutrient broth, BD DIFCO., 8 g/L) inoculated with 3,000 CFU/mL of bacteria in a 50 mL conical tube, 0.015 g of the antibacterial composition was added and suspended (Vortexing), and a sufficiently mixed solution was cultured for 16 hours in a shaking water bath maintained at 35°C, and
after diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = (1 - A_{\text{sample}}/A_{\text{Reference}}) \times 100$$

$$A_{\text{sample}} = \text{Absorbance of medium solution cultured with addition of antibacterial composition}$$

$$A_{\text{Reference}} = \text{Absorbance of medium solution cultured without addition of antibacterial composition}$$

[Advantageous Effects]

Since the antibacterial composition according to some embodiments of the present invention includes a hydrophilic functional group and a hydrophobic functional group at the same time, it is advantageous for imparting antibacterial properties. In addition, the antibacterial property is controlled within a specific range, so that it may provide safe and excellent antibacterial properties.

Since the antibacterial composition according to some embodiments of the present invention has little change in antibacterial activity depending on usage, it may exhibit antibacterial properties within the predicted range even when non-uniformity of concentration occurs unintentionally when applied as a product.

The antibacterial composition according to some embodiments of the present invention may exhibit 80% or more antibacterial properties against specific bacteria.

[Best Mode]

Hereinafter, the present invention will be described in detail.

**<Antibacterial Composition>**

An embodiment of the present invention provides an antibacterial composition including a compound represented by formula 1 below and having an antibacterial activity of 80% or more, as measured by method 1 below, against at least one strain of gram-positive bacteria, gram-negative bacteria, and fungus:

[Formula 1]

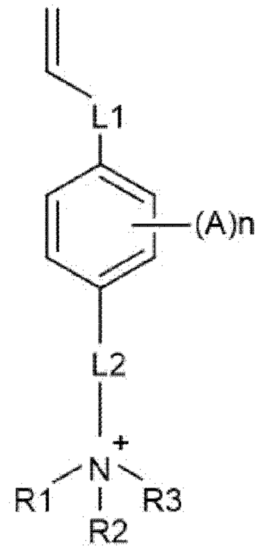

wherein,

L1 and L2 are the same as or different from each other, and each independently a direct bond; a substituted or unsubstituted alkylene group having 1 to 4 carbon atoms; or a substituted or unsubstituted arylene group having 6 to 30 carbon atoms,
A is hydrogen; or a substituted or unsubstituted alkyl group having 1 to 3 carbon atoms,
n is an integer of 0 to 4,
R1 and R2 are the same as or different from each other, and each independently a substituted or unsubstituted alkyl group having 1 to 20 carbon atoms, and at least one of R1 and R2 is a substituted or unsubstituted alkyl group having 1 to 10 carbon atoms, and
R3 is an alkyl group having 4 to 20 carbon atoms, wherein when n is 2 or more, As having n of 2 or more are the same as or different from each other, and
[Method 1]
after putting 25 mL of a broth-type medium (Nutrient broth, BD DIFCO., 8 g/L) inoculated with 3,000 CFU/mL of bacteria in a 50 mL conical tube, 0.015 g of the antibacterial composition was added and suspended (Vortexing), and a sufficiently mixed solution was cultured for 16 hours in a shaking water bath maintained at 35°C, and
after diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = \left(1 - A_{sample}/A_{Reference}\right) \times 100$$

$$A_{sample} = \text{Absorbance of medium solution cultured with addition of antibacterial composition}$$

$$A_{Reference} = \text{Absorbance of medium solution cultured without addition of antibacterial composition}$$

According to an embodiment of the present invention, the hydrogen may be a concept including deuterium ($^2H$) or tritium ($^3H$) as an isotope as well as protium($^1H$).

According to an embodiment of the present invention, the A is hydrogen; or a methyl group.

According to an embodiment of the present invention, the A is all hydrogen.

According to an embodiment of the present invention, at least one of the R1 and R2 is a substituted or unsubstituted alkyl group having 1 to 4 carbon atoms.

According to an embodiment of the present invention, at least one of the R1 and R2 is an unsubstituted alkyl group having 1 to 4 carbon atoms, that is, each independently a methyl group; ethyl group; propyl group; or a butyl group.

According to an embodiment of the present invention, the R3 is an unsubstituted alkyl group having 4 to 20 carbon atoms.

According to an embodiment of the present invention, the R3 is a substituted or unsubstituted alkyl group having 4 to 16 carbon atoms.

According to an embodiment of the present invention, the R3 is an unsubstituted alkyl group having 4 to 16 carbon atoms.

According to an embodiment of the present invention, the R1 to R3 may not be a methyl group or an ethyl group at the same time. In other words, when the R1 and R2 are the same as or different from each other and each independently a methyl group or an ethyl group, the R3 may be an alkyl group having 4 to 16 carbon atoms.

According to an embodiment of the present invention, the R1 to R3 may not be a substituted or unsubstituted alkyl group having 4 to 16 carbon atoms at the same time. In other words, when at least two groups of the R1 to R3 are the same as or different from each other, and each independently an alkyl group having 4 to 16 carbon atoms, the remaining one group (specifically, R1 or R2) may be a methyl group or an ethyl group.

In other words, when R1 to R3 satisfy the above conditions, while the compound represented by formula 1 (quaternary ammonium cation) may electrostatically adsorb to an anion site on the cell surface with respect to at least one strain of gram-positive bacteria, gram-negative bacteria, and fungus, the compound may destroy the surface layer structure of the cell physicochemically by hydrophobic interaction, resulting in efficient killing. Accordingly, the antibacterial activity measured by the aforementioned method 1 may be improved and reached to a certain level or more.

When R1 to R3 do not satisfy the above conditions, in particular, when R1 to R3 are a methyl group or an ethyl group at the same time, or a substituted or unsubstituted alkyl group having 4 to 16 carbon atoms at the same time, the quaternary ammonium cation formed thereby does not sufficiently cause hydrophobic interactions to occur until cell death, so that the antibacterial activity measured by the aforementioned method 1 may be reduced.

According to an embodiment of the present invention, the L1 and L2 are the same as or different from each other, and each independently a direct bond; a substituted or unsubstituted alkylene group having 4 to 16 carbon atoms; or a substituted or unsubstituted arylene group having 6 to 30 carbon atoms.

According to an embodiment of the present invention, the L1 and L2 are the same as or different from each other, and each independently a direct bond; or a substituted or unsubstituted alkylene group having 1 to 4 carbon atoms.

According to an embodiment of the present invention, the L1 is a direct bond.

According to an embodiment of the present invention, the L2 is an unsubstituted alkylene group having 1 to 4 carbon atoms.

According to an embodiment of the present invention, the L2 is a methylene group; or an ethylene group.

According to an embodiment of the present invention, the L2 is a methylene group.

According to an embodiment of the present invention, the compound represented by formula 1 above is any one selected from the group consisting of structures as follows:

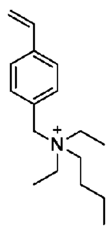
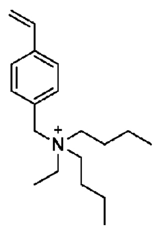
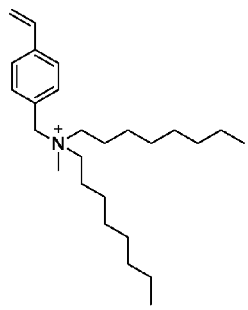
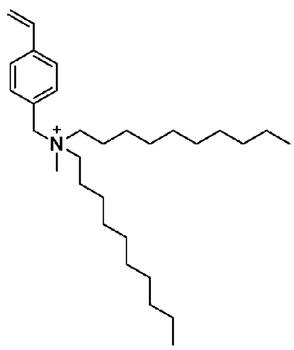

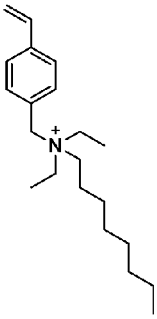
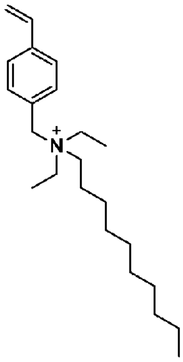
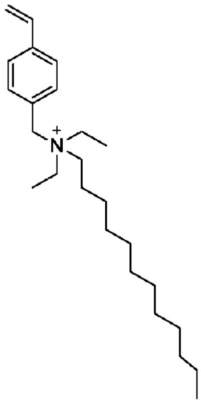
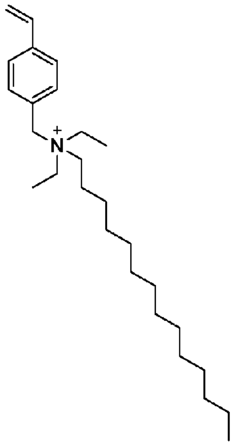

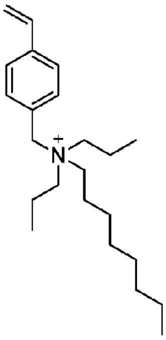
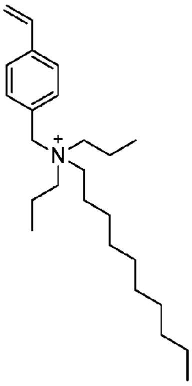
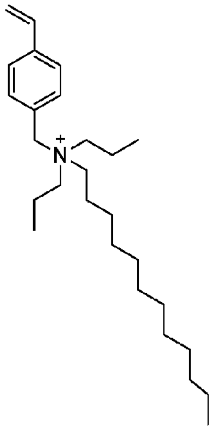
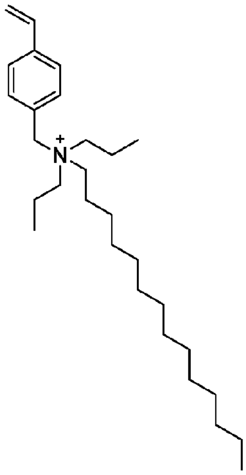

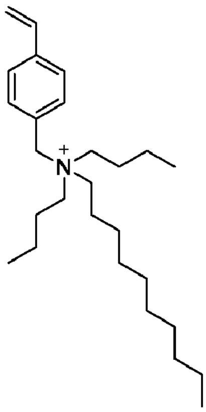.

According to an embodiment of the present invention, the antibacterial activity of the antibacterial composition according to method 1 above may be 80% or more, 85% or more, 90% or more, 93% or more, 95% or more, 97% or more, 98% or more, or 99% or more.

According to an embodiment of the present invention, the bacteriostatic reduction rate (also referred to as antibacterial activity) may be evaluated in the same manner as in method 1 above, except for changing the amount of the antibacterial composition added.

For example, instead of adding 0.015 g of the antibacterial composition, except for adding 0.005 g of the antibacterial composition (method 2 below), adding 0.01 g of the antibacterial composition (method 3 below), or adding 0.02 g of the antibacterial composition (method 4 below), the bacteriostatic reduction rate may be evaluated in the same manner as in method 1 described above.

An embodiment of the present invention may include an antibacterial composition including the compound represented by formula 1 above and having an antibacterial activity of 50% or more, measured by method 2 below, against at least one strain of gram-positive bacteria, gram-negative bacteria, and fungus:

[Method 2]

After putting 25 mL of a broth-type medium (Nutrient broth, BD DIFCO., 8 g/L) inoculated with 3,000 CFU/mL of bacteria in a 50 mL conical tube, 0.005 g of the antibacterial composition was added and suspended (Vortexing), and a sufficiently mixed solution was cultured for 16 hours in a shaking water bath maintained at 35°C.

In addition, after diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = \left(1 - A_{\text{sample}} / A_{\text{Reference}}\right) \times 100$$

$$A_{\text{sample}} = \text{Absorbance of medium solution cultured with addition of antibacterial composition}$$

$$A_{\text{Reference}} = \text{Absorbance of medium solution cultured without addition of antibacterial composition}$$

According to an embodiment of the present invention, the antibacterial activity of the antibacterial composition according to method 2 above may be 50% or more, 55% or more, or 60% or more.

An embodiment of the present invention may include an antibacterial composition including the compound represented by formula 1 above and having an antibacterial activity of 70% or more, measured by method 3 below, against at least one strain of gram-positive bacteria, gram-negative bacteria, and fungus:

[Method 3]

After putting 25 mL of a broth-type medium (Nutrient broth, BD DIFCO., 8 g/L) inoculated with 3,000 CFU/mL of bacteria in a 50 mL conical tube, 0.01 g of the antibacterial composition was added and suspended (Vortexing), and a sufficiently mixed solution was cultured for 16 hours in a shaking water bath maintained at 35°C.

In addition, after diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = \left(1 - A_{\text{sample}} / A_{\text{Reference}}\right) \times 100$$

$$A_{\text{sample}} = \text{Absorbance of medium solution cultured with addition of antibacterial composition}$$

$$A_{\text{Reference}} = \text{Absorbance of medium solution cultured without addition of antibacterial composition}$$

According to an embodiment of the present invention, the antibacterial activity of the antibacterial composition according to method 3 above may be 70% or more, 75% or more, or 80% or more.

An embodiment of the present invention may include an antibacterial composition including the compound represented by formula 1 above and having an antibacterial activity of 90% or more, measured by method 4 below, against at least one strain of gram-positive bacteria, gram-negative bacteria, and fungus:

[Method 4]

After putting 25 mL of a broth-type medium (Nutrient broth, BD DIFCO., 8 g/L) inoculated with 3,000 CFU/mL of bacteria in a 50 mL conical tube, 0.02 g of the antibacterial composition was added and suspended (Vortexing), and a sufficiently mixed solution was cultured for 16 hours in a shaking water bath maintained at 35°C.

In addition, after diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = \left(1 - {A_{\text{sample}}}/{A_{\text{Reference}}}\right) \times 100$$

$$A_{\text{sample}} = \begin{matrix}\text{Absorbance of medium solution cultured} \\ \text{with addition of antibacterial composition}\end{matrix}$$

$$A_{\text{Reference}} = \begin{matrix}\text{Absorbance of medium solution cultured} \\ \text{without addition of antibacterial composition}\end{matrix}$$

According to an embodiment of the present invention, the antibacterial activity of the antibacterial composition according to method 4 above may be 90% or more, 95% or more, or 99% or more.

In an embodiment of the present invention, "having antibacterial properties" means that the antibacterial activity (%) is at least 50%, at least 70% or more, preferably 80% or more, more preferably 90% or more, even more preferably 95% or more, or most preferably 99% or more.

In an embodiment of the present invention, "having antibacterial properties" means that the antibacterial activity (%) by method 1 above is 80% or more, preferably 90% or more, more preferably 95% or more, or most preferably 99% or more.

In an embodiment of the present invention, "having antibacterial properties" means that the antibacterial activity (%) by method 2 above is 50% or more, preferably 55% or more, or most preferably 60% or more.

In an embodiment of the present invention, according to an embodiment, "having antibacterial properties" means that the antibacterial activity (%) of the antibacterial composition by method 3 above is 70% or more, preferably 75% or more, or most preferably 80% or more.

In an embodiment of the present invention, "having antibacterial properties" means that the antibacterial activity (%) by method 4 above is 90% or more, preferably 95% or more, or most preferably 99% or more.

The antibacterial composition according to an embodiment of the present invention has an antibacterial activity of 80% or more, as measured by method 1 above, respectively against at least one strain selected from the group consisting of gram-positive bacteria, gram-negative bacteria and fungus.

In the antibacterial composition having antibacterial activity, the gram-positive bacteria, Gram-negative bacteria and fungal strains may cause various diseases upon contact, and also cause secondary infection. Hence, it is preferable to use one antibacterial agent to exhibit antibacterial properties against all of the gram-positive bacteria, gram-negative bacteria, and fungus.

[Method 1]

After putting 25 mL of a broth-type medium (Nutrient broth, BD DIFCO., 8 g/L) inoculated with 3,000 CFU/mL of bacteria in a 50 mL conical tube, 0.015 g of the antibacterial composition was added and suspended (Vortexing), and a sufficiently mixed solution was cultured for 16 hours in a shaking water bath maintained at 35°C.

After diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an

equation as follows:

$$\text{Antibacterial activity (\%)} = \left(1 - \frac{A_{sample}}{A_{Reference}}\right) \times 100$$

$$A_{sample} = \text{Absorbance of medium solution cultured with addition of antibacterial composition}$$

$$A_{Reference} = \text{Absorbance of medium solution cultured without addition of antibacterial composition}$$

When the antibacterial activity of the antibacterial composition according to an embodiment of the present invention was evaluated by method 1 above, only the case where the antibacterial activity was 90% or more was observed. As a result, it was identified that the antibacterial composition according to an embodiment of the present invention had excellent antibacterial activity.

According to an embodiment of the present invention, the gram-positive bacteria may be any one selected from *Enterococcus faecalis, Staphylococcus aureus, Streptococcus pneumoniae, Streptococcus pyogene, Enterococcus faecium, and Lactobacillus lactis,* but is not limited thereto.

The gram-negative bacteria may be any one selected from *Proteus mirabilis, Escherichia coli, Salmonella typhi, Pseudomonas aeruginosa, Vibrio cholerae, and Enterobacter cloacae,* but is not limited thereto.

According to an embodiment of the present invention, the fungus may be *Candida albicans,* but is not limited thereto.

**<Preparation Method>**

An embodiment of the present invention provides a method for preparing a compound represented by formula 1 below.

According to an embodiment of the present invention, there is provided a method for preparing a compound represented by formula 11 below, wherein the method includes reacting acetonitrile, a compound represented by formula 11 below, and a trialkylamine compound:

[Formula 1]

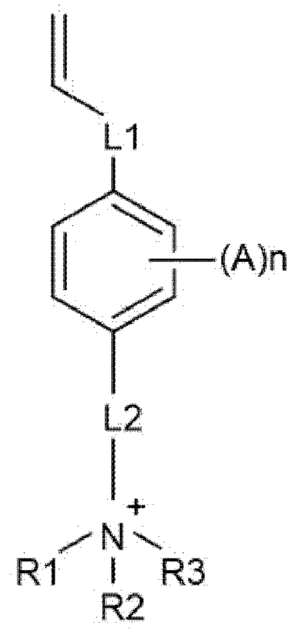

;

and

[Formula 11]

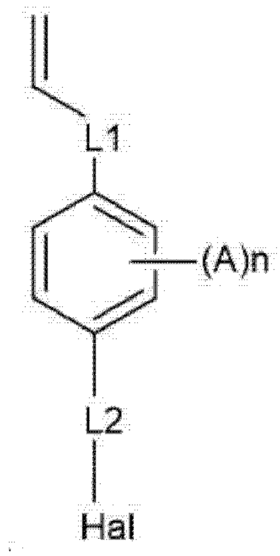

wherein,

L1 and L2 are the same as or different from each other, and each independently a direct bond; a substituted or unsubstituted alkylene group having 1 to 4 carbon atoms; or a substituted or unsubstituted arylene group having 6 to 30 carbon atoms,

A is hydrogen; or an alkyl group having 1 to 3 carbon atoms,

n is an integer of 0 to 4,

R1 and R2 are the same as or different from each other, and each independently a substituted or unsubstituted alkyl group having 1 to 20 carbon atoms, and at least one of R1 and R2 is a substituted or unsubstituted alkyl group having 1 to 10 carbon atoms,

R3 is an alkyl group having 4 to 20 carbon atoms, and
Hal is a halogen group,
wherein when n is 2 or more, As having n of 2 or more are the same as or different from each other.

In this connection, the L1, L2, R1 to R3, A and n may be applied to the aforementioned contents in the antibacterial composition.
According to an embodiment of the present invention, the trialkylamine compound is represented by NR1R2R3. In this connection, R1 to R3 are as defined in formula 1 above.

**<Antibacterial Resin>**

According to an embodiment of the present invention, the antibacterial resin may be a polymer of repeating units derived from one or more compounds represented by formula 1 above included in the aforementioned antibacterial composition.
According to an embodiment of the present invention, the antibacterial resin may be a homopolymer or copolymer of repeating units derived from one or more compounds represented by formula 1 above.
According to an embodiment of the present invention, the antibacterial resin may include a repeating unit derived from another compound in addition to the repeating unit derived from one or more compounds represented by formula 1 above.
Examples of another compound include, but are not limited to, styrene, acrylonitrile, and the like.
The copolymer may be an alternating copolymer, a random copolymer, a block copolymer, or a graft copolymer.
In an embodiment of the present invention, the term "on" that is used to designate a position of one member (layer) with respect to another member (layer) includes both a case that the one member (layer) is adjacent to the another member and a case that any other member (layer) exists between these two members (layers).
In an embodiment of the present invention, when a part is referred to as "including" a component, it means that it may further include other components without excluding other components unless specifically described otherwise.

[Modes for Carrying Out Invention]

Hereinafter, embodiments will be given to describe the present invention in detail. However, the embodiments according to the present invention may be modified in various other forms, and the scope of the present invention is not to be construed as being limited to the embodiments described below. The embodiments of the present invention are provided to more completely explain the present invention to those of ordinary skill in the art.

**<Synthesis Example>**

The reaction scheme of the present disclosure is as follows.

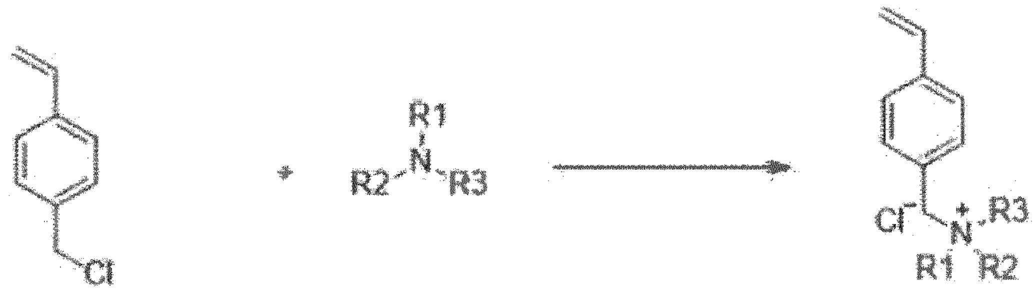

In the above reaction scheme, R1 and R2 are each independently an alkyl group having 1 to 4 carbon atoms, and R3 is an alkyl group having 1 to 20 carbon atoms.

Synthesis Example 1: Synthesis of Compound A

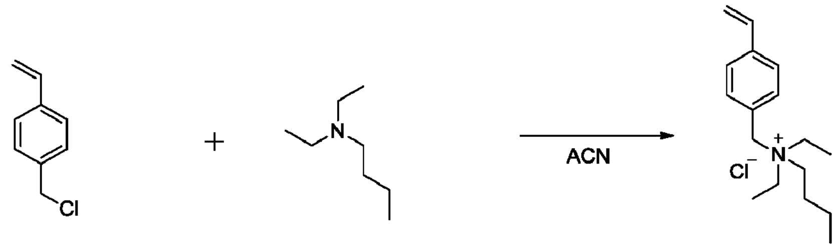


4-Vinylbenzyl chloride *N*,*N*-diethylbutan-1-amine A

After adding 50 mL of acetonitrile (ACN) to a two-neck round-bottom flask and substituting the same with nitrogen, 20 g of 4-vinylbenzyl chloride (1.0eq) and 18.6 g of N,N-diethylbutan-1-amine (1.1eq) were added and reacted overnight while stirring at 45°C. After vacuum drying the organic solvent, hexane was added and stirred for 1 hour. The resulting solid was washed with hexane and filtered to obtain compound A.
$[MS-H]^+ = 246$

**Synthesis Example 2: Synthesis of Compound B**

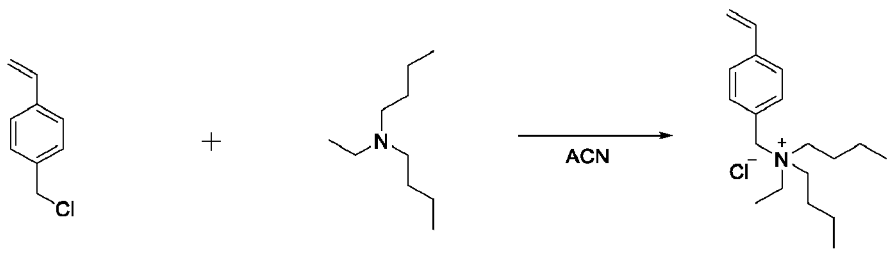


4-Vinylbenzyl chloride *N*-butyl-*N*-ethylbutan-1-amine B

The same method as in Synthesis Example 1 except that N-butyl-N-ethylbutan-1-amine was used instead of N,N-diethylbutan-1-amine was used to obtain compound B.
$[MS-H]^+ = 274$

**Synthesis Example 3: Synthesis of Compound C**

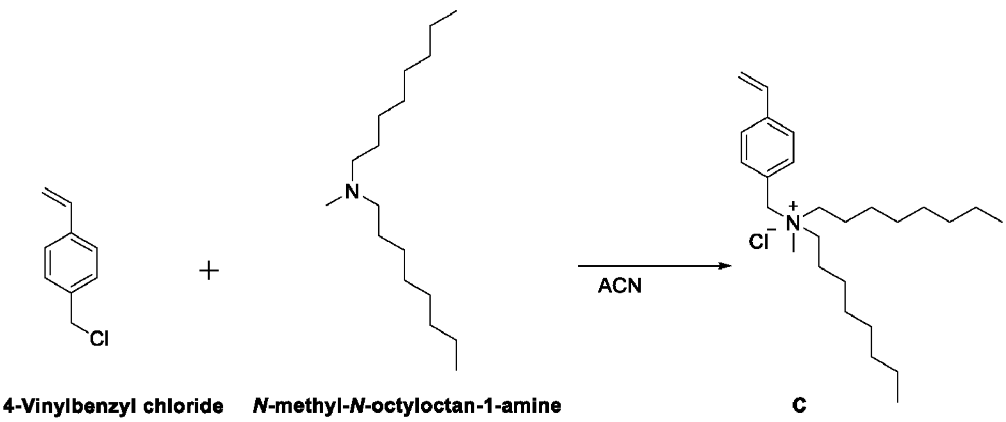


4-Vinylbenzyl chloride *N*-methyl-*N*-octyloctan-1-amine C

The same method as in Synthesis Example 1 except that N-methyl-N-octyloctan-1-amine was used instead of N,N-diethylbutan-1-amine was used to obtain compound C.
$[MS-H]^+ = 372$

**Synthesis Example 4: Synthesis of Compound D**

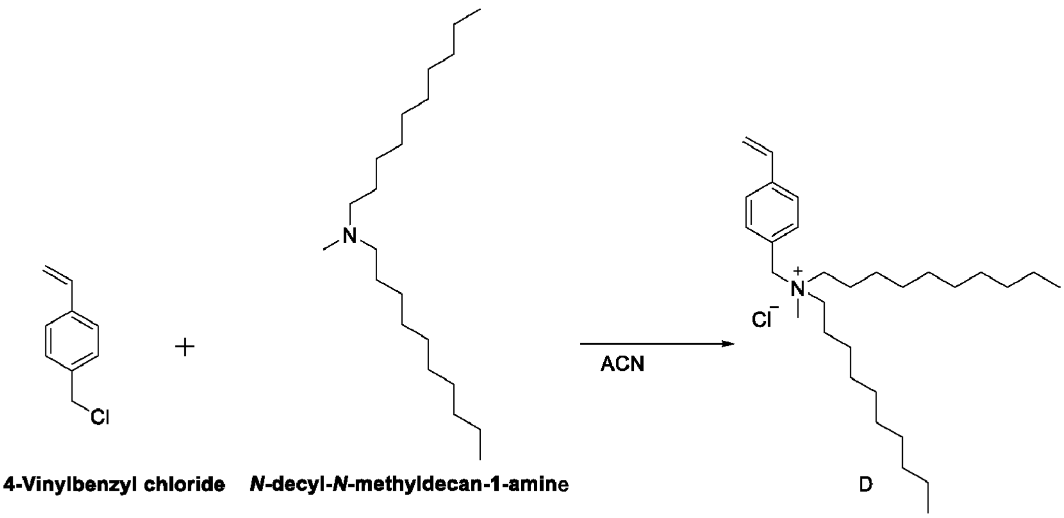


4-Vinylbenzyl chloride *N*-decyl-*N*-methyldecan-1-amine D

The same method as in Synthesis Example 1 except that N-decyl-N-methyldecan-1-amine was used instead of N,N-diethylbutan-1-amine was used to obtain compound D.
$[MS-H]^+ = 428$

**Comparative Synthesis Example 1: Synthesis of Comparative Compound 1**

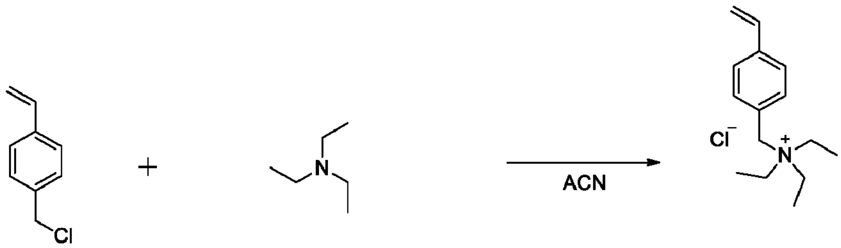


4-Vinylbenzyl chloride Triethylamine Comparative compound 1

The same method as in Synthesis Example 1 except that triethylamine was used instead of N,N-diethylbutan-1-amine was used to obtain comparative compound 1.
$[MS-H]^+ = 218$

**<Examples>**

**Example 1**

The antibacterial activity of the antibacterial composition including the compound A was measured according to method 1 below and is shown in Table 1 below. In this connection, *Proteus mirabilis* (ATCC29906) bacteria was used.

[Method 1]

After putting 25 mL of a broth-type medium (Nutrient broth, BD DIFCO., 8 g/L) inoculated with 3,000 CFU/mL of bacteria in a 50 mL conical tube, 0.015 g of the antibacterial composition was added and suspended (Vortexing), and a sufficiently mixed solution was cultured for 16 hours in a shaking water bath maintained at 35°C.

After diluting the cultured solution to 1/5 using 1× PBS buffer solution, an absorbance (λ=600 nm) was measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = (1 - A_{sample}/A_{Reference}) \times 100$$

$$A_{sample} = \text{Absorbance of medium solution cultured with addition of antibacterial composition}$$

$$A_{Reference} = \text{Absorbance of medium solution cultured without addition of antibacterial composition}$$

**Examples 2 to 4 and Comparative Example 1**

Instead of using the antibacterial composition including Compound A, each antibacterial activity was measured in the same manner as in Example 1 described above, except that the antibacterial composition including each of Compounds B to D and Comparative Compound 1 was independently used. The measurement results of antibacterial activities are shown in Table 1 below.

[Table 1]

| Sample No. | Antibacterial activity (%) | | | |
|---|---|---|---|---|
| | Method 2 (Added amount of antibacterial composition: 0.005g) | Method 3 (Added amount of antibacterial composition: 0.01g) | Method 1 (Added amount of antibacterial composition: 0.015g) | Method 4 (Added amount of antibacterial composition: 0.02g) |
| **Reference** | - | - | - | - |
| **Example 1** | 60.8 | 81.2 | 99.9 | 99.9 |
| **Example 2** | 78.8 | 90.5 | 99.9 | 99.9 |
| **Example 3** | 99.9 | 99.9 | 99.9 | 99.9 |
| **Example 4** | 99.9 | 99.9 | 99.9 | 99.9 |
| **Comparative Example 1** | - | 0.5 | 8.1 | 14.3 |

However, the Reference is a sample to which any antibacterial composition is not added.

In Table 1, Methods 2 to 4 are the same as Method 1 except that the added amount of the antibacterial composition described in Table 1 above is different from one another.

According to Table 1, when the antibacterial activity measured according to method 1 above is compared, the antibacterial compositions of Examples 1 to 4 were all 99.9%, but the antibacterial composition of Comparative Example 1 was 14.3%, indicating that the antibacterial activity of the antibacterial composition including Comparative Compound 1 was significantly lower than the antibacterial activity of the antibacterial composition containing Compounds A to D.

## Claims

1. An antibacterial composition including one or more compounds represented by formula 1 below and having an antibacterial activity of 80% or more, as measured by method 1 below, against at least one strain of gram-positive bacteria, gram-negative bacteria, and fungus:

[Formula 1]

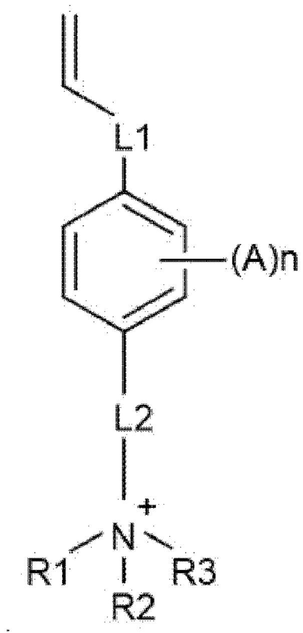

wherein,

L1 and L2 are the same as or different from each other, and are each independently a direct bond, a substituted or unsubstituted alkylene group having 1 to 4 carbon atoms, or a substituted or unsubstituted arylene group having 6 to 30 carbon atoms;
A is hydrogen, or a substituted or unsubstituted alkyl group having 1 to 3 carbon atoms;
n is an integer of 0 to 4;
R1 and R2 are the same as or different from each other, and each independently a substituted or unsubstituted alkyl group having 1 to 20 carbon atoms, and at least one of R1 and R2 is a substituted or unsubstituted alkyl group having 1 to 10 carbon atoms; and
R3 is an alkyl group having 4 to 20 carbon atoms, wherein when n is 2 or more, As having n of 2 or more are the same as or different from each other; and
[Method 1]
after putting 25 mL of a broth-type medium inoculated with 3,000 CFU/mL of bacteria in a 50 mL conical tube, 0.015 g of the antibacterial composition is added and suspended , and a sufficiently mixed solution iss cultured for 16 hours in a shaking water bath maintained at 35°C, and
after diluting the cultured solution to 1/5 using 1× PBS buffer solution, an absorbance is measured using a UV/Vis spectrophotometer, and the measured absorbance is compared with the solution cultured without addition of the antibacterial composition to calculate the antibacterial activity, which is a bacteriostatic reduction rate, by an equation as follows:

$$\text{Antibacterial activity (\%)} = (1 - {A_{sample}}/{A_{Reference}}) \times 100$$

$$A_{sample} = \text{Absorbance of medium solution cultured with addition of antibacterial composition}$$

$$A_{Reference} = \text{Absorbance of medium solution cultured without addition of antibacterial composition}$$

**2.** The antibacterial composition of claim 1, wherein the antibacterial activity is 90% or more.

**3.** The antibacterial composition of claim 1, wherein the gram-positive bacteria are any one selected from *Enterococcus faecalis, Staphylococcus aureus, Streptococcus pneumoniae, Streptococcus pyogene, Enterococcus faecium, and Lactobacillus lactis.*

**4.** The antibacterial composition of claim 1, wherein the gram-negative bacteria are any one selected from *Proteus mirabilis, Escherichia coli, Salmonella typhi, Pseudomonas aeruginosa, Vibrio cholerae, and Enterobacter cloacae.*

**5.** The antibacterial composition of claim 1, wherein at least one of the R1 and R2 is an unsubstituted alkyl group having 1 to 4 carbon atoms.

**6.** The antibacterial composition of claim 1, wherein the R3 is an unsubstituted alkyl group having 4 to 16 carbon atoms.

**7.** The antibacterial composition of claim 1, wherein, when the R1 and R2 are the same as or different from each other and each independently a methyl group or an ethyl group, the R3 is an alkyl group having 4 to 16 carbon atoms.

**8.** The antibacterial composition of claim 1, wherein the L1 is a direct bond.

**9.** The antibacterial composition of claim 1, wherein the L2 is a methylene group.

**10.** The antibacterial composition of claim 1, wherein the compound represented by formula 1 above is any one selected from the group consisting of structures as follows:

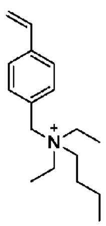 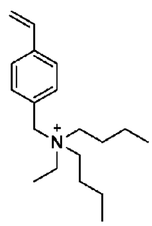 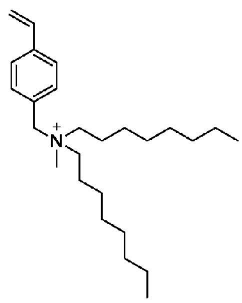 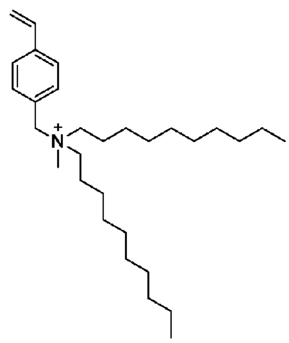

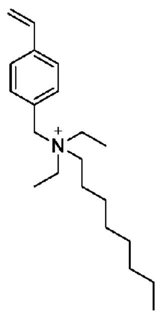 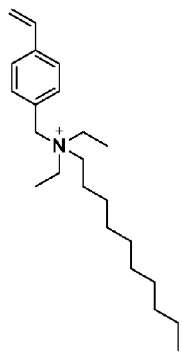 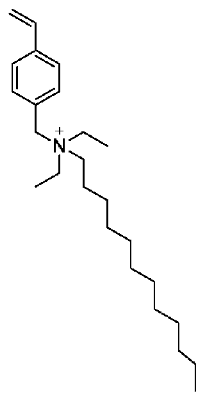 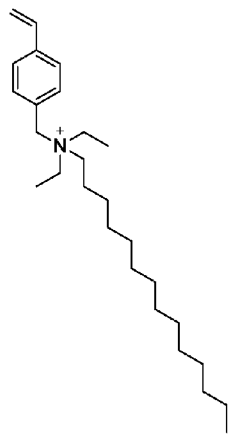

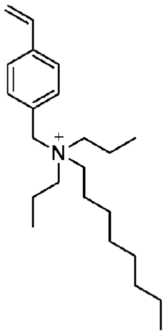
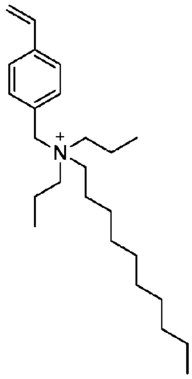
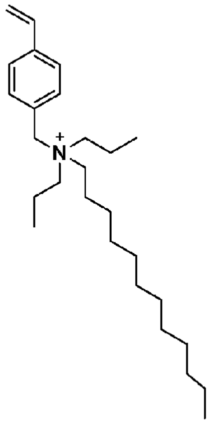
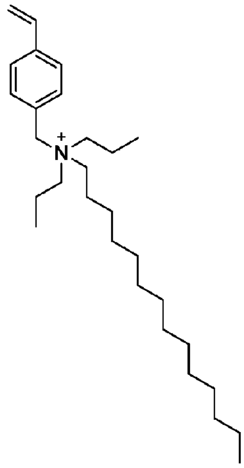

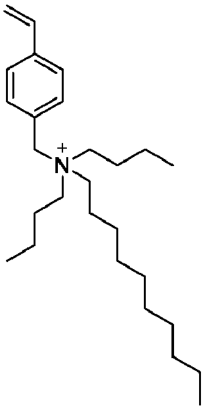
.

## INTERNATIONAL SEARCH REPORT

International application No.
**PCT/KR2022/014549**

**A. CLASSIFICATION OF SUBJECT MATTER**

**A01N 33/12**(2006.01)i; **A01P 1/00**(2006.01)i; **A01P 3/00**(2006.01)i; **C12Q 1/18**(2006.01)i; **C08F 212/08**(2006.01)i; **C08F 220/44**(2006.01)i; **C08F 236/06**(2006.01)i; **C08L 25/06**(2006.01)i; **C08L 25/10**(2006.01)i; **C08L 9/00**(2006.01)i

According to International Patent Classification (IPC) or to both national classification and IPC

**B. FIELDS SEARCHED**

Minimum documentation searched (classification system followed by classification symbols)

A01N 33/12(2006.01); C08F 212/14(2006.01); C08F 220/56(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 항균 조성물(antibacterial composition), 그람 양성균(gram positive bacillus), 그람 음성균(gram negative bacillus), 곰팡이(filamentous fungi)

**C. DOCUMENTS CONSIDERED TO BE RELEVANT**

| Category* | Citation of document, with indication, where appropriate, of the relevant passages | Relevant to claim No. |
|---|---|---|
| X | CN 103613707 A (SHANDONG UNIVERSITY) 05 March 2014 (2014-03-05)<br>See abstract; and claim 1. | 1-10 |
| X | STEVENS, R. et al. Developing antibacterial surgical adhesives: An enhancement of cyanoacrylate polymers. Journal of applied polymer science. 03 February 2021, vol. 138, no. 23, article no. 50538, pp. 1-9.<br>See abstract; and figure 2. | 1-10 |
| X | KANAZAWA, A. et al. Polymeric phosphonium salts as a novel class of cationic biocides. VIII. Synergistic effect on antibacterial activity of polymeric phosphonium and ammonium salts. Journal of applied polymer science. 1994, vol. 53, pp. 1245-1249.<br>See abstract; pages 1248 and 1249, Discussion paragraph; figure 1; and tables 1 and 2. | 1-10 |
| X | SENUMA, M. et al. Antibacterial activity of copolymers of trialkyl (4-vinylbenzyl) ammonium chlorides with acrylonitrile. Die Angewandte Makromolekulare Chemie. 1993, vol. 204, pp. 119-125.<br>See abstract; and tables 1 and 2. | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 January 2023** | **18 January 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2022/014549** |

**C. DOCUMENTS CONSIDERED TO BE RELEVANT**

| Category* | Citation of document, with indication, where appropriate, of the relevant passages | Relevant to claim No. |
|---|---|---|
| X | KOROMILAS, N. D. et al. Synthesis of antimicrobial block copolymers bearing immobilized bacteriostatic groups. Polymer Chemistry. 2016, vol. 7, pp. 3562-3575. See abstract; and formulas 1-6. | 1-10 |

Form PCT/ISA/210 (second sheet) (July 2022)

**INTERNATIONAL SEARCH REPORT**
**Information on patent family members**

International application No.
**PCT/KR2022/014549**

| Patent document cited in search report | Publication date (day/month/year) | Patent family member(s) | Publication date (day/month/year) |
|---|---|---|---|
| CN 103613707 A | 05 March 2014 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210141414
- KR 1020220118143